# EUROPEAN PATENT APPLICATION

(11) **EP 4 756 011 A1**
(43) Date of publication of application: **10.06.2026**
(21) Application number: 24872071.6
(22) Date of filing: 20.09.2024
(51) Int. Cl.: C12N 1/20, A23L 33/135, A61K 8/99, A61K 35/74, A61K 35/741, A61P 1/12, A61P 3/04, A61P 3/10, A61P 37/04

(54) **BACTERIA BELONGING TO GENUS BLAUTIA**

(30) Priority: 25.09.2023 JP 2023161739; 21.06.2024 JP 2024100438
(71) Applicant: Ortho Corporation, Tokyo 105-0021 (JP)
(72) Inventor: SHIRATO Nao, Tokyo 105-0021 (JP); AKASAKA Ryouhei, Tokyo 105-0021 (JP)
(74) Representative: Mewburn Ellis LLP
(86) International application number: PCT/JP2024/033612
(87) International publication number: WO 2025/070280

(57) **Abstract**

There is provided a microbial cell material having exceptional properties. Dead bacterial cells of bacteria belonging to the genus *Blautia* are characterized by being obtained by heat treating bacteria belonging to the genus *Blautia* under acidic conditions. Additionally, the dead bacterial cells of bacteria belonging to the genus *Blautia* are characterized in that the capability thereof to induce production of IL-12, measured through a method in which mouse spleen cells are used, is at least ten times the capability of live bacteria belonging to the genus *Blautia* to induce production of IL-12 under the same conditions. Additionally, a method for producing dead bacterial cells of bacteria belonging to the genus *Blautia* is characterized in that live bacteria belonging to the genus *Blautia* are heat treated under acidic conditions.

## Description

### TECHNICAL FIELD

The present invention relates to a use of bacteria belonging to the genus *Blautia,* and more specifically relates to a use of heat-treated bacterial cells of bacteria belonging to the genus *Blautia.*

### BACKGROUND ART

Through use of lactic acid bacteria supplements, bodily immune strength is improved and health is maintained. For example, Patent Document 1 discloses a preparation of lactic acid bacteria that are formed into nano-sized fine particles and can be used as lactic acid bacteria for applications such as those described above.

However, in recent years, as a result of data analysis involving human subjects, it has become known that bacteria belonging to the genus *Blautia,* which are one type of intestinal bacteria, are inversely related to BMI and risk of diabetes (see Non-patent Document 1).

### Related Art Documents

### Patent Documents

Patent Document 1 Japanese Patent No. 4621218

### Non-patent Documents

Non-patent Document 1 KOJI, Hosomi et al. "Oral administration of Blautia wexlerae ameliorates obesity and type 2 diabetes via metabolic remodeling of the gut microbiota." Nature Communications, 2022, 13:4477.

### DISCLOSURE OF THE INVENTION

### Problems the Invention is Intended to Solve

Use of bacterial cells of bacteria belonging to the genus *Blautia* has not been the subject of extensive study in the prior art.

It is an object of the present invention to provide a microbial cell material having exceptional properties by using bacteria belonging to the genus *Blautia.*

### Means for Solving the Aforementioned Problem

As a result of a variety of studies for the purpose of achieving the aforementioned object, the inventors perfected the present invention upon discovering that a preparation of bacteria belonging to the genus *Blautia* that is prepared under specific conditions exhibits exceptional functionality in terms of improving the environment in the intestine and improving capability to induce production of immunostimulation factors.

Specifically, according to a first aspect, the present invention provides dead bacterial cells of bacteria belonging to the genus *Blautia,* said dead bacterial cells being characterized by being obtained by heat treating bacteria belonging to the genus *Blautia* under acidic conditions.

The aforementioned dead bacterial cells of bacteria belonging to the genus *Blautia* are preferably obtained through heat treatment at a pH of 3.0 to 7.0 and a temperature of 70 to 121°C.

According to a second aspect, the present invention provides dead bacterial cells of bacteria belonging to the genus *Blautia,* said dead bacterial cells being characterized in that the capability thereof to induce production of IL-12, measured through a method in which mouse spleen cells are used, is at least ten times the capability of live bacteria belonging to the genus *Blautia* to induce production of IL-12 under the same conditions.

The aforementioned dead bacterial cells of bacteria belonging to the genus *Blautia* are preferably such that the capability thereof to induce production of IL-10, measured through a method in which mouse spleen cells are used, is at least two times the capability of live bacteria belonging to the genus *Blautia* to induce production of IL-10 under the same conditions.

According to a third aspect, the present invention provides a method for producing dead bacterial cells of bacteria belonging to the genus *Blautia,* said method being characterized in that live bacteria belonging to the genus *Blautia* are heat treated under acidic conditions.

The aforementioned method for producing dead bacterial cells of bacteria belonging to the genus *Blautia* preferably includes performing heat treatment at a pH of 3.0 to 7.0 and a temperature of 70 to 121°C.

### Effect of the Invention

According to the present invention, it is possible to provide a microbial cell material having exceptional properties by using bacteria belonging to the genus *Blautia.*

### BRIEF DESCRIPTION OF THE DRAWINGS

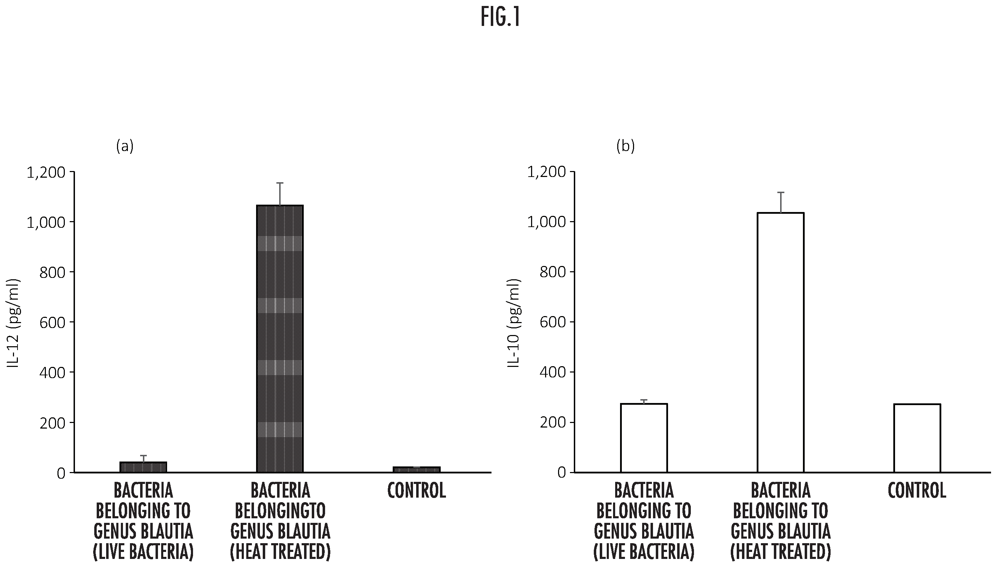

FIG. 1 is a set of graphs showing results from investigating, in test example 1, an influence derived from adding bacteria belonging to the genus *Blautia* (live bacteria or dead bacterial cells) to a culture medium in a culture system of mouse spleen cells, FIG. 1(a) being a graph showing results from investigating capability to induce production of IL-12 in the spleen cells, and FIG. 1(b) being a graph showing results from investigating capability to induce production of IL-10 in the spleen cells;

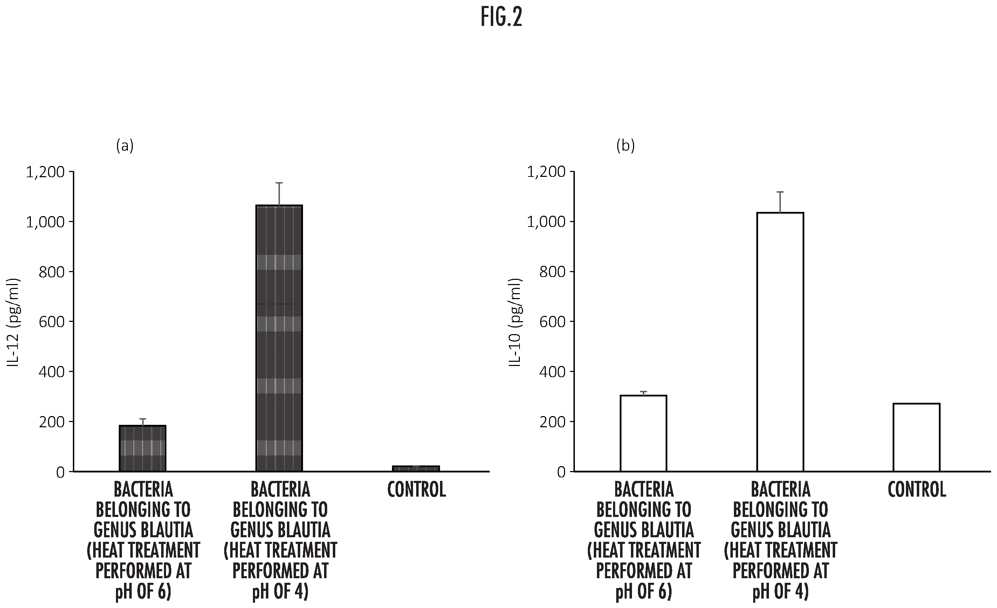

FIG. 2 is a set of graphs showing results from investigating, in test example 1, an influence derived from adding bacteria belonging to the genus *Blautia* (dead bacterial cells in an environment at a pH of 6 or an environment at a pH of 4) to a culture medium in a culture system of mouse spleen cells, FIG. 2(a) being a graph showing results from investigating capability to induce production of IL-12 in the spleen cells, and FIG. 2(b) being a graph showing results from investigating capability to induce production of IL-10 in the spleen cells;

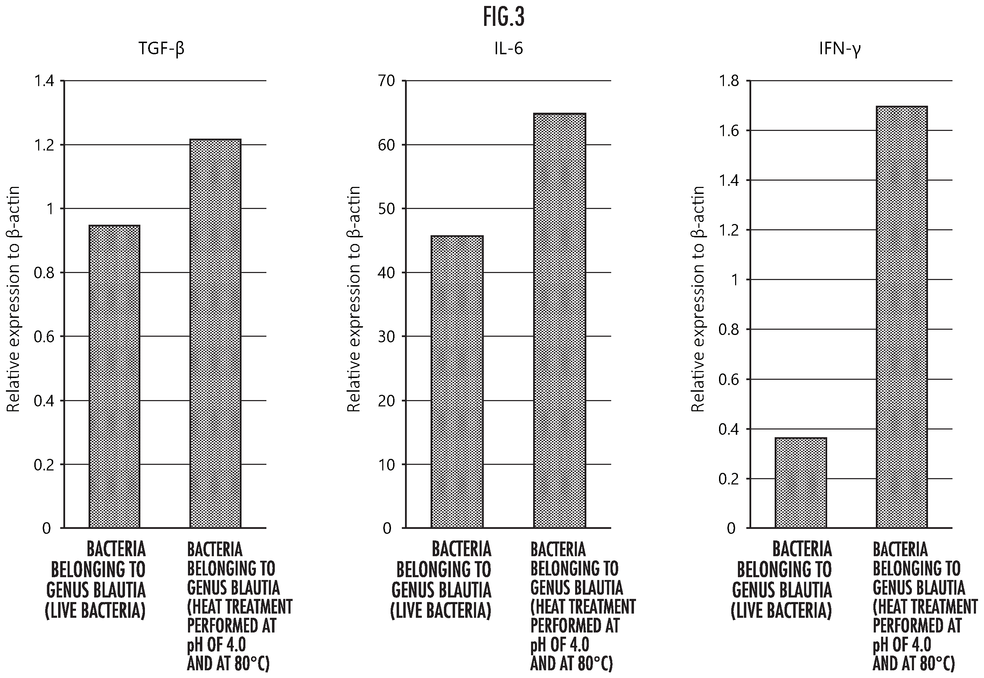

FIG. 3 is a set of graphs showing results from investigating, in test example 2, an influence derived from adding bacteria belonging to the genus *Blautia* (live bacteria, or dead bacterial cells in an environment at a pH of 4.0) to a culture medium in a culture system of mouse spleen cells, said graphs showing results from investigating the cytokine expression level;

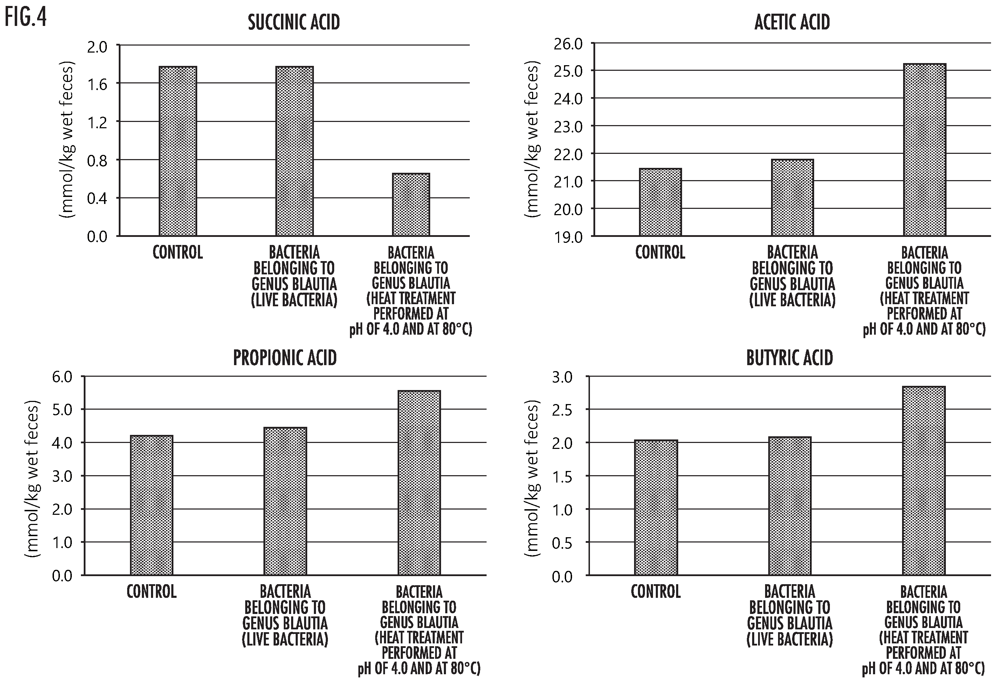

FIG. 4 is a set of graphs showing results from investigating, in test example 2, an influence derived from blending bacteria belonging to the genus *Blautia* (live bacteria, or dead bacterial cells in an environment at a pH of 4.0) into a feed and allowing a BALB/c mouse to freely ingest the feed, said graphs showing results from investigating the concentrations of short-chain fatty acids in the contents of the blind intestine; and

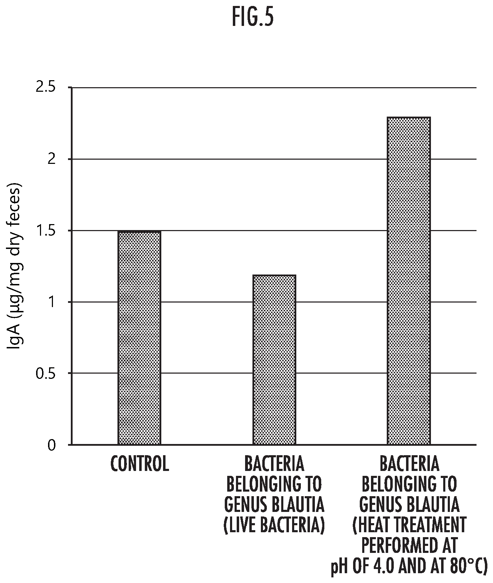

FIG. 5 is a graph showing results from investigating, in test example 3, an influence derived from blending bacteria belonging to the genus *Blautia* (live bacteria, or dead bacterial cells in an environment at a pH of 4.0) into a feed and allowing a BALB/c mouse to freely ingest the feed, said graph showing results from investigating the concentration of IgA in feces.

### MODE FOR CARRYING OUT THE INVENTION

The wording "bacteria belonging to the genus *Blautia*" in the present description refers to bacteria that belong to the genus *Blautia,* which is classified under the phylum *Firmicutes.* Specific examples include *Blautia caecimuris, Blautia glucerasea, Blautia coccoides, Blautia schinkii, Blautia stercoris, Blautia hydrogenotrophica, Blautia faecis, Blautia producta, Blautia hansenii, Blautia luti,* and *Blautia wexlerae.* One of these bacteria species may be used alone, or two or more may be used in combination. Among these species, *Blautia producta* is preferably selected in particular, from the standpoint of an immunostimulation effect.

*Blautia producta* can be exemplified in a preferred manner by *Blautia* strain RD014892 (accession no.: NITE BP-03954, accession date: 01 August 2023) (National Institute of Technology and Evaluation, Patent Microorganisms Depositary, room 122, 2-chome-5-8 Kazusakamatari, Kisarazu, Chiba, Japan, postal code 292-0818), which is deposited at the NITE Patent Microorganisms Depositary (NPMD), or bacteria that are substantially identical to said strain. "Substantially identical" bacteria have the same meaning as would be understood by a person skilled in the art, and would refer, e.g., to bacteria in which the base sequence of a 16SrRNA gene for identifying the genus of bacteria has 98% or higher sequence identity, preferably 99% or higher sequence identity, to the base sequence of a 16SrRNA gene of *Blautia* strain RD014892, and which have the same mycological properties as *Blautia* strain RD014892.

Well-known means can be used in culturing the bacteria belonging to the genus *Blautia,* maintaining the bacterial cells, or the like. Examples of culture media include liquid culture media that contain yeast extracts, peptones, meat extracts, amino acids, salts, minerals, and the like. "Modified GAM" (trade name, modified GAM bouillon, NISSUI CORP.), which is a commercially available culture medium, or the like may also be used. The culturing can be performed by inoculating the culture medium with the bacterial cells and then carrying out stationary culture or aeration-stirring culture at, e.g., 25 to 40°C. For short-term storage, live bacterial cells can be cold-stored while suspended in the culture medium, and for longer-term storage, the live bacterial cells can be suspended in a glycerol solution or another antifreezing solution and then cryogenically preserved.

In preparing the bacteria belonging to the genus *Blautia,* it is possible to prepare a bacterial cell concentrated solution from a state of being a post-culturing culture solution by concentrating the culture solution directly, or by collecting the bacterial cells by means of centrifugal separation, filtration, or the like, furthermore washing the bacterial cells using purified water or the like, and then suspending the bacterial cells in the purified water or the like so as to reach a prescribed bacterial cell concentration. The amount of bacterial cells of the bacteria belonging to the genus *Blautia* within 100 parts by mass of the bacterial cell concentrated solution may be within the range of 0.1 to 50 parts by mass, within the range of 0.5 to 25 parts by mass, or within the range of 1 to 10 parts by mass in terms of dry bacterial cells. A diluent may be incorporated into the bacterial cell concentrated solution. This makes it easier to maintain the properties of the bacterial cells after reconstitution with water, even after the bacterial cells are frozen or freeze-dried.

The diluent is not particularly limited. Examples thereof include: dextrin; maltodextrin; cyclodextrin; xanthan gum; xylitol, sorbitol, maltitol, mannitol, lactitol, and other sugar alcohols; glucose, sucrose, fructose, lactose, dextrose, and other sugars; and adipic acid, citric acid, glutaric acid, succinic acid, tartaric acid, fumaric acid, malic acid, and other organic acids.

According to another aspect, in preparing the bacteria belonging to the genus *Blautia,* it is permissible to implement a pulverization/dispersion process. The pulverization/dispersion process can be carried out by, e.g., pulverizing/dispersing the bacterial cell concentrated solution described above using a means such as stirring, a mixer, a homogenizer, a ball mill, a bead mill, a jet mill, or a generator. In such instances, implementing the pulverization/dispersion process after adding the diluent described above, according to the situation or as needed, makes it possible to prevent re-aggregation of the resulting bacterial cells. In cases where the diluent is incorporated, the amount thereof may be within the range of 1 to 99 mass%, within the range of 10 to 95 mass%, or within the range of 20 to 90 mass% in terms of dry material.

According to yet another aspect, in preparing the bacteria belonging to the genus *Blautia,* it is permissible to implement a dry powderizing process. Examples of a dry powderizing method include techniques such as freeze-drying, vacuum spray drying, and spray drying that utilizes blasts of hot air. Carrying out spray drying that utilizes blasts of hot air ordinarily results in loss of activity of live bacteria and makes it possible to obtain dead bacterial cells thereof.

In the present invention, there is provided a preparation of bacteria belonging to the genus *Blautia* that can be prepared as described above, particularly a preparation obtained through heat treatment under acidic conditions. According to the preparation obtained through such processes, the activity of live bacteria is ordinarily lost and dead bacterial cells of the bacteria belonging to the genus *Blautia* are obtained, making it possible to minimize variations in quality that are associated with live bacteria. Additionally, as indicated in the examples, which shall be described later, the dead bacterial cells of the bacteria belonging to the genus *Blautia* have the capability to induce production of immunostimulation factors (IL-12, IL-10, TGF- β, IL-6, IFN-γ, etc.) in immune cells, and have functionality for improving the intestinal environment and inducing production of IgA, which is an immunizing molecule. Thus, the present invention can be suitably used as, e.g., an active ingredient of a functional composition for improving bodily immune strength and maintaining health. In particular, the present invention can be suitably used in, *inter alia,* a functional food for maintaining health in a healthy individual. According to another aspect, the present invention makes it possible to provide a functional material, or a contributing component thereof, that can be used in a health food or supplement for promoting health.

The heat treatment of the bacteria belonging to the genus *Blautia* can be carried out using, e.g., a jacket tank, a thermostatic tank, or an autoclave either directly on the post-culturing culture solution or, as necessary, after the culture solution has been prepared into the bacterial cell concentrated solution described above. There are no limitations as to the pH in such instances; the pH may be, e.g., within the range of 3.0 to 7.0, within the range of 3.0 to 6.0, or within the range of 3.0 to 5.0. The temperature for the heat treatment is not limited, but may be, e.g., within the range of 70 to 121°C, within the range of 80 to 110°C, or within the range of 80 to 100°C. The heat treatment time is not limited, but may be, e.g., within the range of 30 minutes to 120 hours, within the range of 30 to 90 minutes, or within the range of 30 to 60 minutes. If processing in such an environment is insufficient, the immunostimulation capabilities and other desired performance will tend to suffer, which is undesirable.

In discerning whether the bacteria belonging to the genus *Blautia* provided by the present invention have been subjected to sufficient processing in the environment described above, the preparation history of said bacteria may be confirmed. Alternatively, this matter can be discerned from the standpoint of functionality according to the situation. For example, this matter can be discerned from the standpoint of functionality such as whether the capability of dead bacterial cells of said bacteria to induce production of IL-12, measured through a method in which mouse spleen cells are used, is at least ten times the capability of live bacteria belonging to the genus *Blautia* to induce production of IL-12 under the same conditions, or whether the capability of dead bacterial cells of said bacteria to induce production of IL-10, measured through a method in which mouse spleen cells are used, is at least two times the capability of live bacteria belonging to the genus *Blautia* to induce production of IL-10 under the same conditions.

The dead bacterial cells of the bacteria belonging to the genus *Blautia* provided by the present invention can be used in the form of various products, such as foods/beverages, functional foods, pharmaceuticals, cosmetics, and animal feeds, as desired.

There is no particular limitation as to the type of food/beverage into which the present invention is blended. For example, the present invention can be blended into staple foods, supplementary foods, confectionery, and seasonings, such as: coffee, fruit juices, soft drinks, beer and other alcoholic beverages, milk, miso soup, soups, black tea, teas, powdered beverages, nutritional agents, syrups, margarine, pastes, jams, and other liquid (fluid) foods; and cooked rice, bread, potato products, mochi, *furikake,* ham, sausages, candy, chocolate, gum, gummies, snack foods, baked confectionery, and other solid foods. The present invention may be molded into the form of a powder, granules, tablets, or the like, in accordance with the application. Additionally, the present invention can be blended with a diluent, a filler, a binder, a thickener, an emulsifier, a colorant, a perfume, a food additive, a seasoning, or the like, as needed.

In addition to a food to be supplied for human consumption, the present invention can be prepared as a feed imparted with functionality by mixing the dead bacterial cells of the bacteria belonging to the genus *Blautia* into a raw material of the feed in advance in cases where the dead bacterial cells are to be mixed into the feed and administered to livestock, pets, or other animals. Specifically, the present invention can be used as a functional feed by using the dead bacterial cells of the bacteria belonging to the genus *Blautia* as an active ingredient and adding said active ingredient to a feed for: pigs, chickens, cows, horses, sheep, and other livestock; pets (dogs, cats, birds); and sea bream, young Japanese amberjack, tuna, eel, puffer fish, and other fish.

Examples of functional foods include supplements, health drinks, health foods, dietary supplements, functional health foods, functional nutritional foods, designated health foods, function-labeled foods, and materials for addition to foods. The forms of these products are not particularly limited; for example, it is possible for the products to be provided as tablets, capsules, granular agents, powders, or beverages.

As a pharmaceutical, the present invention can be combined with, *inter alia,* a pharmacologically permitted base material, as appropriate, and the resulting combination can be used as a pharmaceutical preparation. The pharmaceutical preparation can be provided in such forms as, e.g., tablets, chewable agents, capsule agents, granular agents, powders, pills, syrups, tinctures, decoctions, and solutions.

As a cosmetic, the present invention can be combined with, *inter alia,* a pharmacologically permitted base material, as appropriate, and the resulting combination can be used as a cosmetic preparation. Examples include lotions, toners, creams, milky lotions, powders, foundations, packs, gels, jellies, aerosols, soaps, cleansing foams, bath liquids, body soaps, sun protection products, salves, patches, and adhesive plasters.

As described above, the dead bacterial cells of the bacteria belonging to the genus *Blautia* provided by the present invention can be configured in various forms as probiotic materials and functional materials. In such instances, other components can be incorporated in addition to the bacteria of the genus *Blautia.* Examples of the other components include microbial cell materials, particularly dead bacterial cell materials, of bacteria belonging to the genus *Lactobacillus,* the genus *Lacticaseibacillus,* the genus *Lactiplantibacillus,* the genus *Lactococcus,* the genus *Bifidobacterium,* the genus *Streptococcus,* the genus *Enterococcus,* the genus *Akkermansia,* the genus *Christensenella,* the genus *Clostridium,* the genus *Bacteroides,* the genus *Bacillus,* the genus *Paraprevotella,* the genus *Faecalibacterium,* the genus *Lentilactobacillus,* the genus *Eubacterium,* and the genus *Veillonella.*

As described above, the dead bacterial cells of the bacteria belonging to the genus *Blautia* provided by the present invention can be configured in various forms as probiotic materials and functional materials. In such instances, the amount of the dead bacterial cells of the bacteria belonging to the genus *Blautia* is to be determined, as appropriate, in consideration of the relationship between the amount used in each form and the effective amount for exhibiting functionality when the various forms are employed. Typically, the amount of the dead bacterial cells of the bacteria belonging to the genus *Blautia,* in terms of dry material, may be within the range of 0.001 to 100 mass%, within the range of 0.001 to 50 mass%, or within the range of 0.001 to 10 mass%. Additionally, the amount thereof in terms of number of bacteria may be within the range of 2.0 × 10⁷ to 2.0 × 10¹² cells/g, within the range of 2.0 × 10⁷ to 1.0 × 10¹² cells/g, or within the range of 2.0 × 10⁷ to 2.0 × 10¹¹ cells/g.

In cases where humans are to ingest the dead bacterial cells of the bacteria belonging to the genus *Blautia* provided by the present invention, the administered amount is to be set, as appropriate, in accordance with the health and age of a given subject, or with the degree of functionality required. Typically, the injection amount of lactic acid bacteria, in terms of dry material, may be within the range of 0.0005 to 500 mg/day/kg wt., within the range of 0.005 to 50 mg/day/kg wt., or within the range of 0.05 to 5 mg/day/kg wt. Additionally, the amount in terms of number of bacteria may be within the range of 1.0 × 10⁶ to 1.0 × 10¹² cells/day/kg wt., within the range of 1.0 × 10⁷ to 1.0 × 10¹¹ cells/day/kg wt., or within the range of 1.0 × 10⁸ to 1.0 × 10¹⁰ cells/day/kg wt. Examples

The present invention is more specifically described below by way of examples. However, the scope of the present invention is in no way limited to the scope of the provided examples.

### [Preparing samples]

*Blautia* strain RD014892 was used as the bacteria belonging to the genus *Blautia.* The bacteria were cultured in MRS culture medium at 37°C for 24 hours in an anaerobic environment. The resulting culture solution was centrifuged at 8000 × g for 10 minutes, a supernatant was removed, and then a substance obtained by adding distilled water to the culture solution and suspending bacterial cells therein was retrieved as a sample (live bacteria). Moreover, the same culture solution was adjusted to a pH of 6.0 or a pH of 4.0 by adding acetic acid thereto and then heat treated at 80°C for 30 minutes, after which a substance obtained by similarly collecting the bacterial cells and suspending the same in distilled water was retrieved as a heat-treated sample (pH 6.0 or pH 4.0).

### [Test example 1]

### (Method)

A spleen was harvested from a BALB/cA mouse (female, 10 weeks old) in accordance with common practice. The spleen was harvested in a sterile state, to the extent possible, within a clean bench. Cells were collected from the harvested spleen using a cell strainer (pore size: 100 µm), after which the cells were prepared in a liquid culture medium to reach a cell concentration of 2.5 × 10⁶ cells/mL. The liquid culture medium that was used was prepared by blending FBS (THERMO FISHER SCIENTIFIC) at a final concentration of 10% and Penicillin-Streptomycin-Neomycin (PSN) Antibiotic Mixture (THERMO FISHER SCIENTIFIC), as appropriate, in RPMI-1640 (L-glutamine; contains phenol red; FUJIFILM WAKO PURE CHEMICAL INDUSTRIES). Bacteria belonging to the genus *Blautia* (live bacteria, heat treatment performed at a pH of 6.0, or heat treatment performed at a pH of 4.0) were added to the resulting cell solution to reach a final concentration of 1.0 µg/mL in terms of dry bacterial cells, and culturing was carried out at 37°C in a 5% CO₂ environment. The cytokine concentrations of respective post-culturing supernatants were measured using ELISA at a point in time 24 hours from the start of culturing for IL-12 and at a point in time 96 hours from the start of culturing for IL-10. The average values and standard deviations for these measurements were calculated from six wells for IL-12 and from five wells for IL-10. Additionally, wells of only spleen cells to which no bacteria belonging to the genus *Blautia* were added were used as a control.

### (Evaluation)

As shown in FIG. 1, with the live bacteria belonging to the genus *Blautia* both IL-12 and IL-10 were produced in roughly the same amounts as in the control to which no bacterial cells were added, whereas these immunostimulation factors were produced in substantially greater amounts with the dead bacterial cells of the bacteria belonging to the genus *Blautia* obtained through heat treatment.

Additionally, as shown in FIG. 2, the capability of dead bacterial cells of the bacteria belonging to the genus *Blautia* to induce production of the immunostimulation factors was particularly significant in cases where the dead bacterial cells were heat treated in an environment at a pH of 4.0.

### [Test example 2]

### (Method)

A spleen cell solution obtained from a BALB/cA mouse (female, 10 weeks old) was prepared in the same manner as in test example 1. Bacteria belonging to the genus *Blautia* (live bacteria, or heat treatment performed at a pH of 4.0 and at 80°C) were added to the cell solution to reach a final concentration of 1.0 µg/mL in terms of dry bacterial cells, , and culturing was carried out at 37°C in a 5% CO₂ environment. The cells were collected after six hours had elapsed from the start of culturing, and RNA was extracted therefrom using an RNeasy Mini Kit (QIAGEN). cDNA was then synthesized from the resulting RNA using a ReverTra Ace qPCR RT Master Mix (TOYOBO). The mRNA expression levels for various cytokines (TGF-β, IL-6, IFN-γ) were measured through real-time PCR using the synthesized cDNA and iTaq Universal SYBR Green Supermix (BIO-RAD), and the resulting measurement values were standardized according to the mRNA expression level for β-actin. Moreover, similar real-time PCR was performed separately using spleen cells to which no bacteria belonging to the genus *Blautia* were added, and relative values of the mRNA expression levels for the various cytokines were calculated with reference to the result thereof.

### (Evaluation)

As shown in FIG. 3, in the case where the bacteria belonging to the genus *Blautia* (heat treatment performed at a pH of 4.0 and at 80°C) were added, there was found to be an increase in the gene expression levels for various cytokines (TGF-β, IL-6, IFN-γ) that are known to be immunostimulation factors over the case where the bacteria belonging to the genus *Blautia* (live bacteria) were added.

### [Test example 3]

### (Method)

BALB/c mice were acclimatized for one week and then allowed, for one week, to freely ingest a refined feed (AIN-93G) into which the respective samples were blended so as to reach the administered amounts indicated in the table below. In-colon feces were then collected from the euthanized mice, IgA was measured using ELISA, and the concentrations of short-chain fatty acids (succinic acid, acetic acid, propionic acid, butyric acid) in the contents of the blind intestine were measured.

**[Table 1]**

| | Administration amount (number of bacteria · individual/day) |
|---|---|
| Control | (Purified water) |
| Live bacteria | 1 × 10⁹ |
| Heat treatment performed at pH of 4.0 and at 80°C | 1 × 10⁹ |

### (Evaluation)

### •Concentrations of short-chain fatty acids in contents of blind intestine

As shown in FIG. 4, in the group administered bacteria belonging to the genus *Blautia* (heat treatment performed at a pH of 4.0 and at 80°C), there was found to be an increase in acetic acid, propionic acid, and butyric acid, which are known to have functionality for improving the intestinal environment, over both the control group and the group administered bacteria belonging to the genus *Blautia* (live bacteria). However, there was found to be a decrease in succinic acid, which is known to cause diarrhea and the like.

### •Concentration of IgA in feces

As shown in FIG. 5, in the group administered bacteria belonging to the genus *Blautia* (heat treatment performed at a pH of 4.0 and at 80°C), there was found to be an increase in IgA, which is an immunizing molecule, over both the control group and the group administered bacteria belonging to the genus *Blautia* (live bacteria).

From the foregoing, it was made clear that heat treating bacteria belonging to the genus *Blautia* under acidic conditions enhances the functionality for improving the environment in the intestine.

## Claims

1. Dead bacterial cells of bacteria belonging to the genus *Blautia,* said dead bacterial cells being **characterized by** being obtained by heat treating bacteria belonging to the genus *Blautia* under acidic conditions.

2. The dead bacterial cells of bacteria belonging to the genus *Blautia* according to claim 1, wherein the dead bacterial cells are obtained through heat treatment at a pH of 3.0 to 7.0 and a temperature of 70 to 121°C.

3. Dead bacterial cells of bacteria belonging to the genus *Blautia,* said dead bacterial cells being **characterized in that** the capability thereof to induce production of IL-12, measured through a method in which mouse spleen cells are used, is at least ten times the capability of live bacteria belonging to the genus *Blautia* to induce production of IL-12 under the same conditions.

4. The dead bacterial cells of bacteria belonging to the genus *Blautia* according to claim 3, wherein the capability thereof to induce production of IL-10, measured through a method in which mouse spleen cells are used, is at least two times the capability of live bacteria belonging to the genus *Blautia* to induce production of IL-10 under the same conditions.

5. A method for producing dead bacterial cells of bacteria belonging to the genus *Blautia,* said method being **characterized in that** live bacteria belonging to the genus *Blautia* are heat treated under acidic conditions.

6. The method for producing dead bacterial cells of bacteria belonging to the genus *Blautia* according to claim 5, wherein the method includes performing heat treatment at a pH of 3.0 to 7.0 and a temperature of 70 to 121°C.
